# EUROPEAN PATENT SPECIFICATION

(11) **EP 1 117 458 B1**
(45) Date of publication and mention of the grant of the patent: **18.02.2009**
(21) Application number: 99952160.2
(22) Date of filing: 01.10.1999
(51) Int. Cl.: A61M 25/01

(54) **GUIDEWIRE CAPTURE DEVICE**
FÜHRUNGSDRAHTFANGVORRICHTUNG
DISPOSITIF DE CAPTURE DE FIL-GUIDE

(30) Priority: 02.10.1998 AU PP633898
(43) Date of publication of application: 25.07.2001
(73) Proprietor: Endogad Research Pty Limited, Parramatta, NSW 2150 (AU)
(72) Inventor: WHITE, Geoffrey, H., East Balmain, NSW 2041 (AU); YU, Weiyun, Birchgrove, NSW 2041 (AU)
(74) Representative: Baker, Colin John
(86) International application number: PCT/AU1999/000848
(87) International publication number: WO 2000/020064

(56) References cited:
- US-A- 4 873 978
- US-A- 4 873 978
- US-A- 5 112 310
- US-A- 5 360 443
- US-A- 5 634 475
- US-A- 5 693 086

## Description

The present invention relates to a device and a method for capturing a guidewire being progressed along the lumen of a bodily duct. More particularly the invention relates to such a device that is positioned at the end of a catheter and is adapted to be positioned within a bodily duct and is adapted to capture a guidewire being progressed through the bodily duct from the other direction and to direct it into the catheter.

### Background Art

It is well known that through disease, arteries of humans are susceptible to the development of distended sacs known as aneurysms which are susceptible to rupture. Traditionally, aneurysms have been treated by radical surgical intervention. This approach is risky for the patient and is, in many cases, not feasible due to other pre-existing disease states in the patient. More recently there have been a number of proposals for the intraluminal placement of an intraluminal graft bridging the aneurysms and thereby isolating an active arterial duct from the aneurysmal sac. One such arrangement is described in PCT patent publication WO 95/08966.

Difficulties arise in the placement of such intraluminal grafts when the aneurysm extends from a single artery into one or more divergent arteries. In this case a so called "trouser graft" must be used. In such a graft a single tubular body bifurcates into two smaller tubular bodies. The intention being that the single tubular body is placed in the single artery and the two smaller tubular bodies are respectively placed in the two divergent arteries (for example, see closest prior art document US Patent No. 5,360,443 to Barone). In practice it has proven very difficult to effectively place a trouser graft.

It is known to place a bifurcated graft in the single artery that either has two short tubular extensions or has one long tubular extension and one short tubular extension. In the former case, the whole of this initial graft is positioned within the single artery and further grafts are inserted in an upstream direction through each of the divergent arteries and respectively connected to one of the tubular extensions of the initial graft. In the latter case, the initial graft is placed in the single artery with the long extension positioned in the ipsi-lateral one of the divergent arteries. In this case a separate graft is introduced upstream through the contra-lateral one of the divergent arteries and is connected to the short extension of the initial graft. In either of these procedures, there is difficulty in getting the graft that has been introduced in an upstream direction through a divergent artery to join up with the appropriate tubular extension of the initial graft. One approach to doing this is to cause a guidewire to proceed up one of the divergent arteries into the initial graft and then to turn and proceed down the other tubular extensions into the other of the divergent arteries. This guidewire can then be used to guide the separate graft or grafts into connection with the tubular extension or extensions of the initial graft.

In the above methods of placement of a bifurcated graft, and in other similar methods that involve the passage of a guidewire up one leg of the graft and down the other, there is a difficulty in ensuring that the guidewire comes down the correct vessel so that it may be captured adjacent to the patient's skin through a cut down or an arterial puncture. In the case of a bifurcated graft placed in the aorta, for instance, the graft extensions will extend into the iliac arteries. The guidewire used in positioning the contralateral limb of the graft into a respective one of the iliac arteries will need to be directed down the iliac artery into the femoral artery. If the guidewire is not very accurately directed it can become diverted away from the femoral artery into the internal iliac artery: a position from which it is not readily accessed.

The present invention relates to a new catheter that can be used to facilitate the capture of a guidewire passed through a bifurcated intraluminal graft in an artery.

### Disclosure of the Invention

The present invention consists in a guidewire capture sheath for use in a bodily vessel comprising:
(a) a catheter; and
(b) a capture means housed by, connected to, or integral with the catheter; at least a portion of the capture means being capable of expanding and collapsing between a first condition and at least a second condition, wherein when in the second condition, the capture means is adapted to guide a guidewire into the catheter.

In a preferred embodiment, when a portion of the capture means is in the first condition, it is contracted to a cross-sectional area that is smaller than that of the catheter; and when in the second condition, it is expanded to a cross-sectional area that is larger than that of the catheter.

A method for guiding a guidewire which is extending in a first vessel into a catheter is described, the method comprising:
(a) causing or allowing at least one portion of a guidewire capture sheath according to the first aspect of the invention to assume its first, contracted, condition;
(b) introducing the guidewire capture sheath according to (a) into the first vessel or a second vessel which communicates with the first vessel;
(c) advancing the guidewire capture sheath such that the capture means is directed towards the guidewire;
(d) causing or allowing the at least one portion of the capture means to assume its second, expanded, condition; and
(e) causing the guidewire and the capture means to approach one another, such that the guidewire is guided into the catheter by the capture means.

In a preferred embodiment, the capture means may be disposed within the catheter when it is in the first, contracted, condition; and be caused to assume the second, expanded, condition upon being projected from the catheter. Alternatively, the capture means may be permanently disposed to project from one end of the catheter. In one embodiment according to the latter case, the capture means is preferably configured so that when in the first, contracted, condition, it will form a substantially conical nose on the catheter that will assist the passage of the catheter through the bodily vessel. In a third alternative, the capture means may be intermediate the ends of the catheter.

In some embodiments, it is preferable to introduce the guidewire capture sheath into the vessel with the guidance of a guidewire. In such embodiments, as the guidewire is inserted before the catheter of the guidewire capture sheath, it is preferable to provide an orifice within the capture means through which the guidewire may pass. Accordingly, when in its first, collapsed, condition, the capture means of these embodiments defines a guidewire following aperture. As the catheter will be threaded over the distal end of this placement guidewire outside the body, it is only necessary that the guidewire following aperture be just sufficient to allow the catheter to be slid along the placement guidewire. Once the catheter has been placed in the vessel in which it is to capture a second guidewire, the capture means can be expanded to its second condition. In this second condition, it is preferable that the capture means expands to a size at which its outer parameter bears against the luminal wall of the vessel. In preferred embodiments, such expansion provides assurance that as the guidewire and the guidewire capture sheath are caused to approach one another, the guidewire will eventually make contact with the capture means, and be funnelled into the catheter. Typically, the guidewire will be projected down the catheter until its free end is located outside the body. The catheter can then be withdrawn from the vessel leaving the guidewire projecting from the vessel. A new catheter, such as a graft deploying catheter, can then be guided up the guidewire that has been captured by the guidewire capture sheath according to this invention.

There are a wide variety of forms that the capture means may take. In a first form, at least a portion of the capture means comprises a number of petal like members disposed around the circumference of one end of the catheter. Each petal like member is connected to its neighbouring petal like members by a web of a thin flexible film. In preferred embodiments, the petal like members are pivotable between a first position, in which they are close together at their free ends, as are the petals in a flower bud, and a second position, in which they are spread apart, as in the manner of an opened flower. In the latter position, the petal like members and the webs together form a funnel that will direct a guidewire into the lumen of the catheter. In this embodiment of the invention, the petal like members may be caused to move between their first and second positions by a wire or wires connected to the radially outer side of one or more of the petal like members, such that by pulling on the wire or wires the petal like members are caused to be moved from their first position to their second position. Alternatively, the petal like members may be formed as two superposed leaves connected at their free ends, an outer one of the leaves being connected at its base to the catheter and the inner end being connected at its base to a tube disposed within the catheter. In this arrangement relative movement between the catheter and the tube will move the petal like members between their first and second positions. In a preferred form, a number of the petal like members are at least semi-permeable to allow for the passage of blood.

Also note that in embodiments of the invention where at least a portion of the capture means is comprised of a plurality of petal like members, the materials and/or construction of at least that portion of the capture means may be such that no manipulative intervention is required to cause the petal like members to go from their first position to their second position. An example of such embodiments includes cases where the capture means is formed of a material which has the capacity to "memorise" a particular shape, and is, therefore, capable of memory-aided change. Such a material has a continuous tendency to return to that shape following any event which causes it to be temporarily deformed. A capture means with at least a portion being comprised of a plurality of petal like members will, according to one such embodiment, be initially manufactured such that its petal like members are preformed in their respective second - open - positions. Prior to introduction of the guidewire capture sheath into a vessel, such a capture means is then manually compressed and pushed into the lumen of the catheter, such that its petal like members are caused to assume their respective first, closed, positions. Once the guidewire capture sheath is in the appropriate position within the vessel, the capture means is caused to extend beyond an end of the catheter. Such extension results in a release of the petal like members from the confines of the catheter lumen, thereby allowing them to spring back into their respective second, open, positions. When the petal like members are each in such a position, together, they preferably provide a funnel capable of directing a guidewire into the lumen of the catheter.

In further preferred embodiments, wherein at least a portion of the capture means is comprised of a plurality of petal like members, the capture means is formed of a material which is capable of changing its shape when subjected to a change in temperature and is, therefore, capable of heat-aided change. In this case, the capture means will, upon introduction into a patient, undergo an increase in temperature caused by its placement within the body of the patient. It will, consequently, have its petal like members change from their first, closed, position to their second, open, position. In such embodiments, it may be preferable, before using the invention, to predetermine the desired greatest cross-sectional diameter of the petal like members when in their second, open, positions, so that the radial size of this greatest cross-sectional diameter is appropriate to the circumstances of the particular case. In embodiments of the invention wherein temperature changes are employed to cause the capture means to assume the appropriate position for "capturing" a guidewire, materials such as Nitinol are preferably used in the manufacture of the capture means.

Naturally, in all of the embodiments so far described, wherein the capture means with petal like members is formed of a "memory" material or a heat sensitive material, it may not be necessary to provide a means, such as a wire, which the surgeon must manipulate in order to cause the capture means to change from a first condition to a second condition. In these embodiments, such change may occur without manipulative intervention.

In another embodiment of the invention, at least a portion of the capture means comprises a section of the catheter adjacent one end of the catheter. In this case, a short section of the catheter is formed as a number of flexible, longitudinally extending wires. For part of their length distal to the end of the catheter, the wires are surrounded by an elastic sheath. In this arrangement, when a wire extending along the lumen of the catheter and connected to the end of the catheter adjacent the capture means is pulled, the wires will be caused to bow radially outwardly. This outward bowing of the wires will, in turn, cause the elastic sheath to be belled outwardly against the luminal wall of a vessel in which the guidewire capture sheath is deployed. As the guidewire approaches, and comes into contact with, the capture means, it will be guided into the lumen of the catheter.

Also note that the discussion relating to the use of a "memory" materials, and in particular, that relating to the use of materials which respond to changes in temperature, for the formation of the capture means with a plurality of petal like members is, in appropriate circumstances, equally applicable to the formation of a capture means which is essentially comprised of a plurality of wires as described above. Naturally, where such a material is used, there may be no need to provide a means to enable a surgeon to cause the capture means to change from a first condition to a second condition, as such change will occur independently.

In a further preferred embodiment with a similar mechanism of action to that where the capture means is formed of a plurality of wires, a plurality of fine slots are formed in a wall of the catheter leaving a plurality of fine strips of catheter therebetween. In such an embodiment, the slots are preferably formed such that they are each parallel to a longitudinal axis of the catheter, and circumferentially spaced apart around the catheter. In the preferred form, the slots are all of the same length, wherein said length is a function of at least one of the designated dimensions (eg, the length) of the capture means. A similar mechanism to that described above, such as a wire, upon which a surgeon can pull, is employed by this embodiment to cause the capture means to change from a first condition to a second condition.

In yet another embodiment of the invention, at least a portion of the capture means comprises a helical spring that is surrounded by an elastic sheath. In preferred arrangements of this embodiment, when in a relaxed state, the spring has a greater diameter at its free end than at an end which is connected to or integral with the catheter. In alternative arrangements of this embodiment, however, the spring may also have a constant diameter along its length when in the relaxed state. In either case, or for arrangements in which the diameter of the spring varies along its length, it is preferable that when in the relaxed state, the spring has, at least at one point, a diameter which is greater than the diameter of the catheter in which it is initially housed. Indeed, according to these embodiments, the spring is in the first condition housed within the catheter which holds it in a retracted condition. If a push rod extending along the catheter is used to push the free end of the spring out of the catheter (thereby allowing the spring to assume a relaxed state), the free end of the spring, and the elastic sheath attached to it, will then assume a second, expanded, condition. In this condition, the free end of the spring will engage against the luminal wall of a vessel in which the guidewire capture sheath has been deployed, and will direct a guidewire with which it comes into contact into the catheter.

As above, it is noteworthy that the discussion relating to the use of a "memory" materials, or those which respond to changes in temperature, for the formation of the capture means with a plurality of petal like members is, in appropriate circumstances, equally applicable to the formation of a capture means which is essentially comprised of a spring.

In a still further embodiment, the capture means includes a distal portion of the first end of the catheter which, in at least its second condition forms a funnel member. The capture means of this embodiment is most preferably formed of either a shape "memory" material or a spring material. It may, however, also be formed of a material, such as Nitinol, which is capable of changing shape upon being subjected to a change in temperature. In embodiments where the capture means is formed of the "memory" material, the catheter is housed within an outer sheath, and upon movement of the catheter relative the outer sheath, the capture means is caused to change from a first condition, in which it forms a part of the first end of the catheter, to a second condition, in which it forms the funnel member. In this second condition, the open end of the funnel member engages the luminal wall of a vessel in which the guidewire capture sheath has been deployed, and will direct a contra-directed guidewire into the catheter.

In similar embodiments, where the capture means is formed of a temperature sensitive material, such as Nitinol, there may, of course, be no need to provide an outer sheath as described above. This is because the capture means, according to this particular embodiment, will remain in its first, contracted, condition until such time as its temperature has increased to a point at which it changes to a second, expanded, condition.

While there are many operative procedures that could use the guidewire capture sheath according to the present invention, it is particularly useful in the procedures described in PCT patent specifications PCT/AU97/00046 and PCT/AU96/00714.

### Brief Description of the Drawings

By way of example only, preferred embodiments of the invention are provided below with reference to the accompanying drawings, in which:
Fig. 1 is a diagrammatic partially cut-away ventral view of a patient with an aortic aneurysm which has been bridged by an intraluminal graft using the guidewire capture sheath according to the present invention;
Fig. 2 is a side elevational view of one embodiment of a tubular intraluminal graft for use in the procedure described with reference to Fig. 1;
Fig. 3 is a longitudinal diametrical sectional view through the intraluminal graft of Fig. 2;
Fig. 4 is a detailed elevational view of one end of the intraluminal graft of Fig. 2;
Fig. 5 is a detailed perspective view of the first end of the intraluminal graft of Fig. 4 showing how the alternate crests of the end wire of the graft are pushed radially outward during insertion of the graft;
Figs. 6 and 6a are vertical sectional views of two embodiments of possible bifurcated grafts mounted over delivery catheters for use in carrying out the present method;
Figs. 7a to 7i show the stages of carrying out a procedure for the placement of a bifurcated graft in a patient using the guidewire capture sheath according to the present invention;
Figs. 8a and 8b respectively show the capture means of a first embodiment of a guidewire capture sheath according to this invention in its contracted and expanded conditions;

### Preferred Mode of Carrying out the Invention

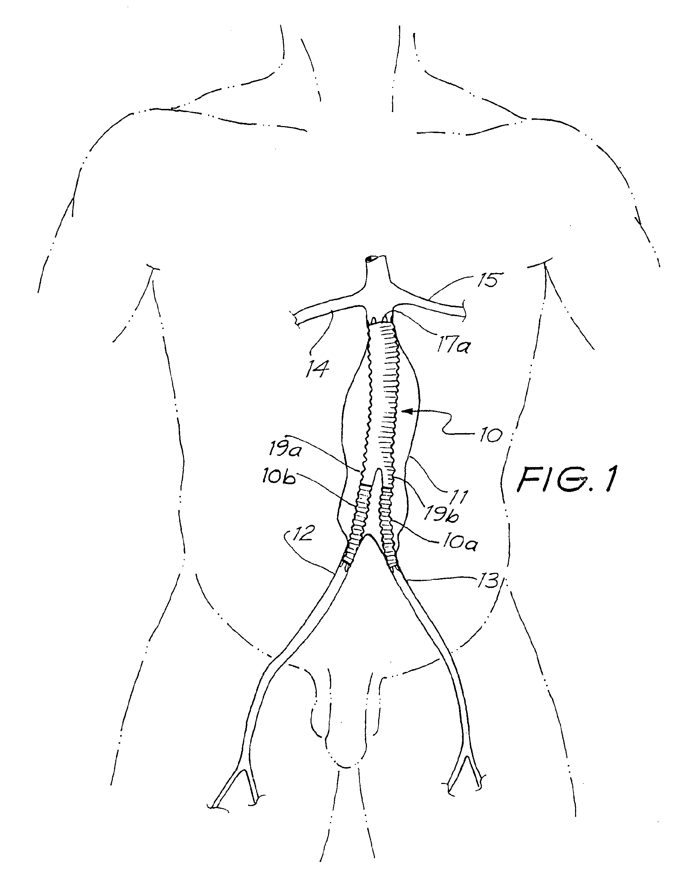

A bifurcated or trouser graft comprising the three intraluminal grafts 10, 10a and 10b is adapted for insertion transfemorally into a patient to achieve bridging and occlusion of an aortic aneurysm extending into the left and right iliac arteries. As is seen in Fig. 1 the aorta 11 is connected to the left and right iliac arteries 13, 12. The aortic aneurysm is located between the renal arteries 14 and 15 and the iliac arteries 12, 13 with the aneurysm extending down the iliac arteries 13, 12.

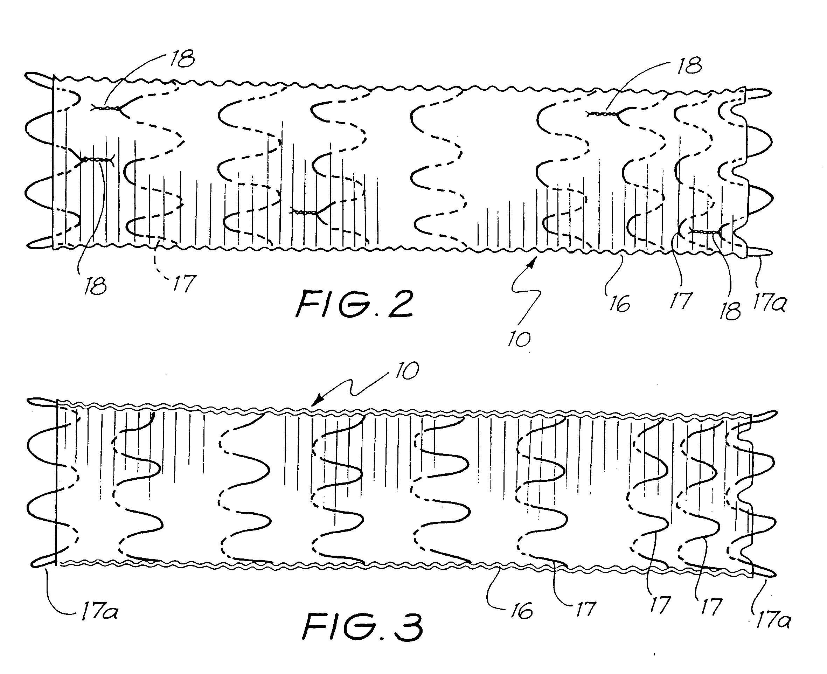

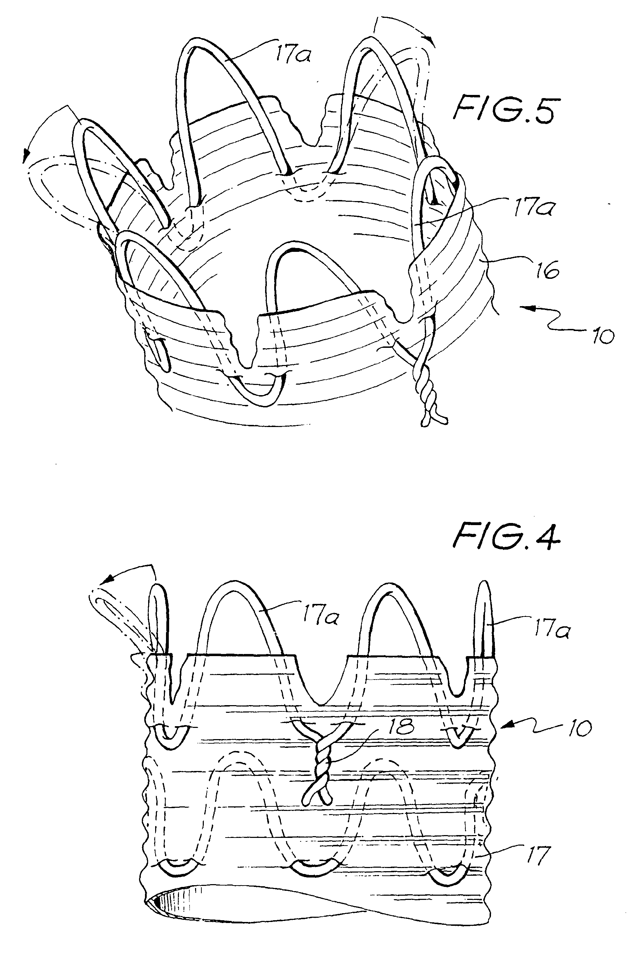

Each intraluminal graft (as is shown in Figs. 2-5) can comprise a crimped tube 16 of woven polyester. Other materials could be utilised including polytetrafluoroethylene, polyurethane and composites thereof. The tube 16 is reinforced along its length by a number of separate and spaced apart stainless-steel wires 17 (each of which can have the depicted generally closed sinusoidal shape). The wires 17 are preferably as thin as possible and are typically 0.3 to 0.4 mm in diameter. The wires 17 are malleable and may be bent into any desired shape. In preferred embodiments, the wires 17 are not resilient to any substantial extent so that they have to be physically expanded into contact with the aorta rather than expanding by virtue of their own resilience. The wires 17 are each woven into the fabric of the tube 16 such that alternate crests of each wire 17 are outside the tube 16 with the remainder of that wire 17 inside the tube 16 (except in the case of the endmost wires 17 as will be hereinafter described). The ends of each wire 17 are located outside the tube 16 and are twisted together to form a tail 18. While the ends are depicted as twisted together to form a tail 18, the ends can also be crimped together. The tails 18 of alternate wires 17 are bent to extend in opposite longitudinal directions along the outside surface of the tube 16.

The endmost wires 17a overhang the respective ends of the tube 16 so that alternate crests of those wires 17a extend longitudinally beyond the end of the tube 16. The endmost wires 17a preferably have an amplitude of about 6 mm and a wavelength such that between six and eight crests are spaced around the circumference of a 22 mm diameter graft. The next two adjacent wires 17 preferably are spaced as close as possible to the endmost wire 17a and respectively have amplitudes of 4 mm and 5 mm. These wires will typically have the same wavelength initially as the endmost wire 17a. Thereafter, throughout the graft the wires 17 are spaced at 15 mm intervals, have an amplitude of 6 mm, and have substantially the same initial wavelength as the endmost wire 17a.

As the aneurysm extends beyond the branching of the iliac arteries 12 and 13 from the aorta 11 a single tubular graft is insufficient to bridge the aneurysm while maintaining blood flow to each of the iliac arteries 12, 13. Rather than using a single tubular graft, in the present method three separate tubular grafts 10, 10a and 10b are used. The downstream end of a first one of the grafts 10 is provided with a bifurcation to form a pair of short tubular graft extensions 19a, 19b of the graft 10. The short tubular graft extensions 19a, 19b may be passively expandable by blood flow or actively expandable by balloon expansion or by spring self-expansion.

The graft portions 10a, 10b which are adapted to extend into the respective iliac arteries 13, 12 each have an upstream end having a common diameter. The upstream ends interlock with the respective extensions 19a, 19b of the graft 10 which, itself, is adapted to be positioned within the aorta 11. Preferably, this interlocking is achieved by balloon-expansion or spring self-expansion of the upstream ends such that there is a frictional engagement between the respective upstream ends and the extensions 19a, 19b.

In addition to having a straight cylindrical tube, the diameter of the downstream end of the graft portions 10a, 10b can be provided in varying diameters so as to suit the diameter of the iliac artery into which graft portions 10a, 10b are being implanted.

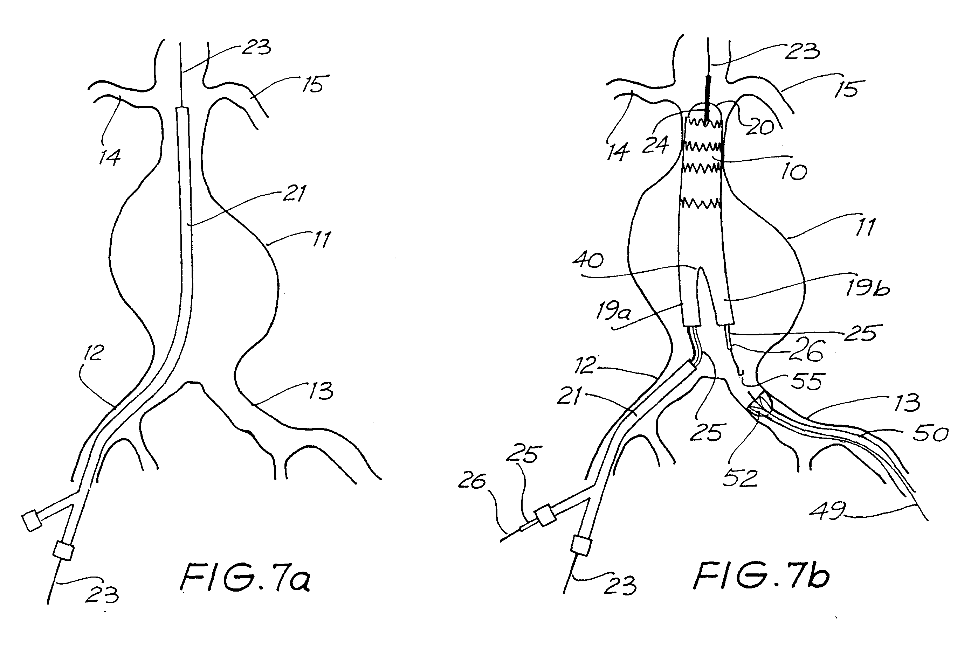

The method for positioning the intraluminal graft will now be described with reference to Figs. 7a - 7i. In carrying out the method, an incision or puncture is made to expose one of the femoral arteries (eg: ipsilateral), which flows from the corresponding iliac artery. Then, using the Seldinger needle technique, a 0.035" diameter floppy tipped flexible guidewire is inserted into and through the femoral artery, through the iliac artery 12, and into the aorta 11, such that it traverses the aneurysm. An 8 French haemostatic sheath is then introduced over the wire to control bleeding. An angiographic catheter is introduced to allow an angiogram to be taken of the patient to show the position of the renal arteries 14, 15, and other relevant anatomical structures in the patient.

The floppy tipped flexible guidewire is then withdrawn and an Amplatz extra stiff (AES) guidewire 23 (0.035" diameter) is passed through the angiographic catheter into the aorta 11 (see Fig. 7a). After withdrawal of the angiographic catheter, the stiff guidewire 23 is left *in situ*. A catheter sheath 21, preferably of 24 French, and trocar are then introduced into the aorta 11 over the stiff guidewire 23 (see Fig. 7a); and a balloon catheter 24 introduced into the sheath 21.

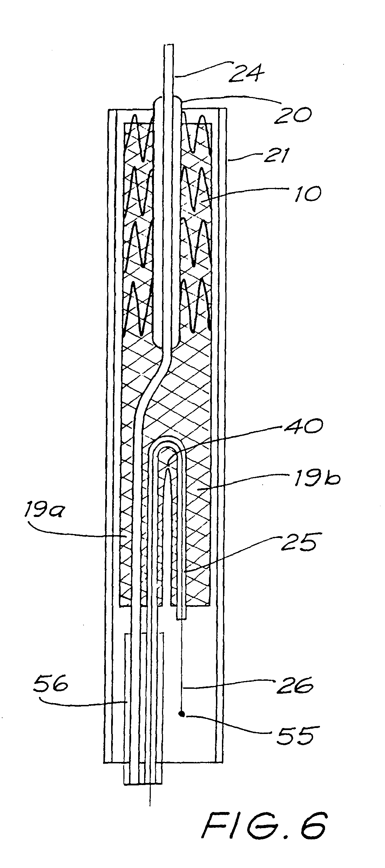

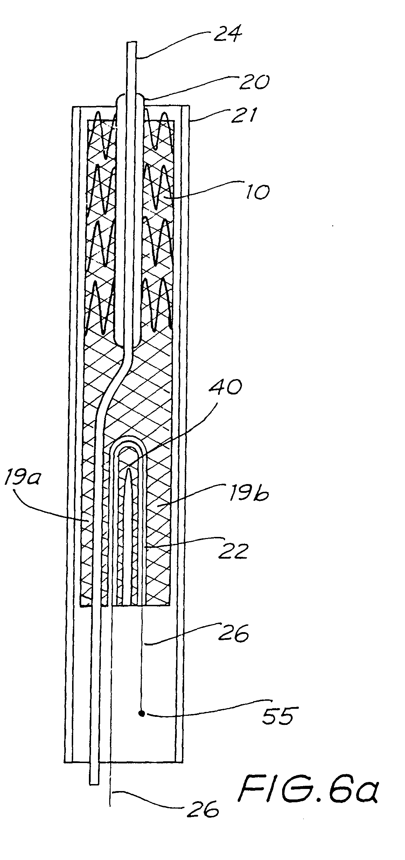

As depicted in more detail in Fig. 6, in a preferred embodiment, the balloon catheter 24 is a delivery catheter which is pre-packaged with a bifurcated graft 10; this graft having first and second tubular graft extensions 19a, 19b separated at a bifurcation point 40, and a thin catheter 25 containing a multi-directional guidewire 26 extending in a first direction through the first tubular extension 19a and then in a second different direction through the second graft extension 19b.

The balloon catheter 24 and thin catheter 25 can be linked together in a common catheter sheath 56 which serves to better ensure correct positioning of the thin catheter 25 and multidirectional guidewire 26 on placement of the graft 10 in the vessel. In addition to being slidable through the tubular graft extensions 19a, 19b, the thin catheter 25 can be fixed in place in the graft 10 prior to insertion of the graft 10 into a vessel. The thin catheter 25 can be sutured, glued, woven or secured in any appropriate manner into the body of the graft 10.

While the multidirectional guidewire 26 is depicted in Fig. 6 inside a thin catheter 25, the invention envisages arrangements in which no such catheter is required. In an alternative arrangement depicted in Fig. 6a, for example, the multidirectional guidewire 26 is positioned within a tubular channel 22 formed in the body of the graft 10. The channel 22 serves to ensure that the multidirectional guidewire 26 remains in the desired position in the first and second tubular graft extensions 19a, 19b for a desired period.

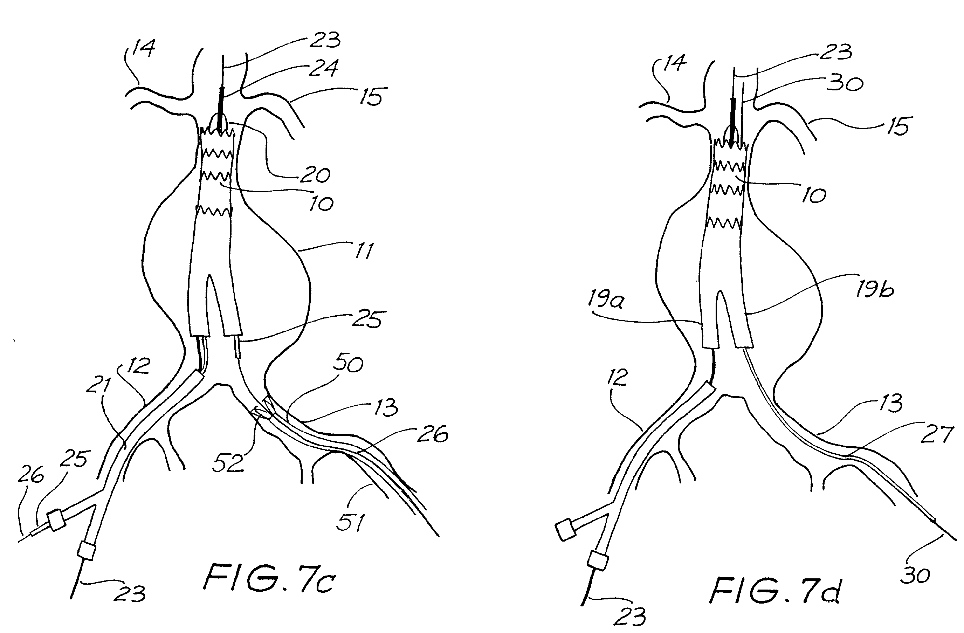

When the balloon catheter 24 is positioned within the aorta 11 at the desired position, the sheath 21 is partially withdrawn to free the graft 10, and the balloon 20 is inflated (see Fig. 7b). The purpose of inflating the balloon 20 at this time is to engage an upstream end of the first graft 10 with the aorta wall; upstream of the aneurysm but downstream of the renal arteries 14, 15. In preferred embodiments, the first graft 10 is of such a length that the short tubular graft extensions 19a, 19b are disposed wholly within the aorta 11. The balloon 20 is then deflated but the balloon catheter 24 is left in place for the time being (see Fig. 7c). Deflation of the balloon 20 will allow blood to flow down the graft 10, thereby distending each of the tubular graft extensions 19a, 19b.

The thin catheter 25 is preferably 3 French. The multidirectional guidewire 26 is preferably of a non-kinking material so that it may be extended relative to the thin catheter 25 in a downstream direction. The multidirectional guidewire 26 may have at its tip a small hook 55. The multidirectional guidewire 26 is preferably comprised of a Nitinol core having a hydrophilic coating.

An incision or puncture is then made in the contralateral femoral artery and a contralateral guidewire 49 is inserted into that artery and advanced into the contralateral iliac artery 13 above the internal iliac artery. A guidewire capture sheath 50 is positioned over the contralateral guidewire 49 with its free end also positioned in the contralateral iliac artery 13 above the internal iliac artery (see Fig. 7b). The guidewire capture sheath 50 is comprised of a catheter 51 having at its free end a capture device 52 that will be described in more detail later in this specification. Once the guidewire capture sheath 50 is positioned in the contralateral iliac artery 13 the contralateral guidewire 49 is removed and the capture device 52 expanded into contact with the inside surface of the wall of the iliac artery 13.

The multidirectional guidewire 26 may now be advanced down the contralateral iliac artery 13 and it will contact the upstream surface of the capture device 52 and be directed down the lumen of the catheter 51 until the end 55 of the multidirectional guidewire 26 is outside the body of the patient (see Fig. 7c). The capture device may now be fully or partially collapsed and the guidewire capture sheath 50 withdrawn.

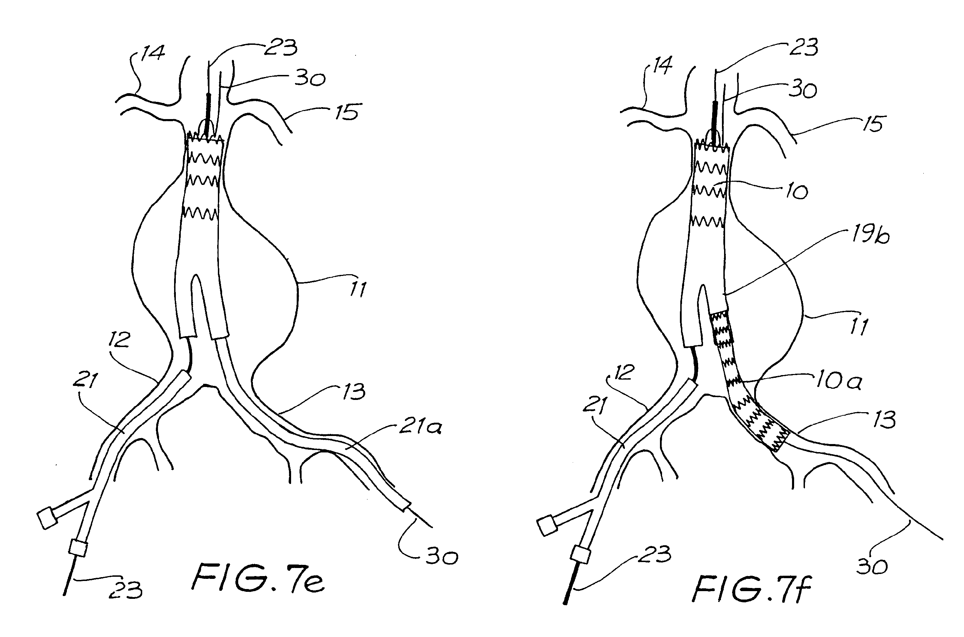

Once the multidirectional guidewire 26 is retrieved, the thin catheter 25 is then withdrawn via the ipsilateral side and another catheter 27 is fed through the contralateral femoral artery up the multidirectional guidewire 26 until it is within the first graft 10 and reaches at least to the top of the second tubular extension 19b (see Fig. 7d). The guidewire 26 is then withdrawn and a thicker stiff guidewire 30 inserted through the contralateral femoral artery into the catheter 27. The catheter 27 is then removed and a catheter sheath 21a, preferably of 24 French, and trocar are introduced over the stiff guidewire 30 (see Fig. 7e).

Prior to extending the multidirectional guidewire 26 into the contralateral iliac and femoral arteries, a catheter sheath (that can be similar to catheter sheath 21) can be extended upstream through the contralateral femoral and iliac arteries to reduce any tortuosity that may be present in these arteries and so facilitate guiding of the multidirectional guidewire 26 therethrough.

A second balloon catheter on which is packaged a second tubular graft 10a, is then introduced through catheter sheath 21a until its upper end is well within the second tubular extension 19b and within the iliac artery 13 at its lower end. The balloon on the catheter is inflated such that the upper end of graft 10a is frictionally engaged with the second tubular extension 19b (see Fig. 7f). The inflation of the balloon on the second balloon catheter supports the graft 10a during the withdrawal of the first balloon catheter 24 through the ipsilateral artery 12. Then the balloon on the catheter is deflated and the catheter maintained in place to provide continued support for the grafts 10, 10a in the aorta 11 while the third graft 10b is positioned.

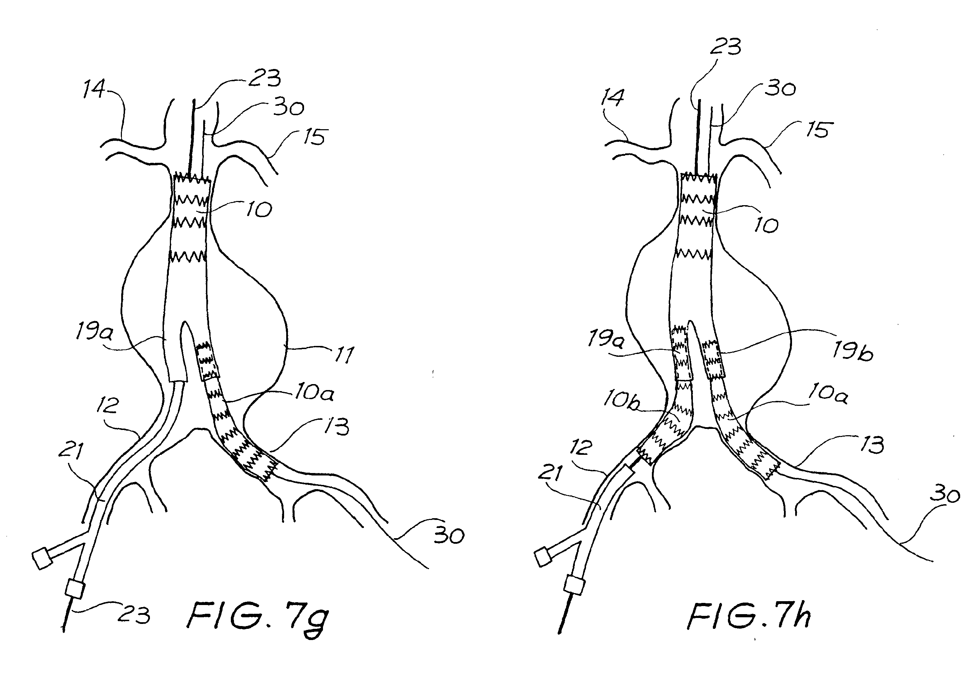

The catheter sheath 21a is then removed (see Figs. 7f and 7g) and a third balloon catheter on which is packaged a tubular graft 10b (the third balloon catheter and graft 10b can be identical to that used to deploy graft 10b) is introduced into the sheath 21 on guidewire 23. It is advanced until its upstream end is within the first tubular extension 19a and, following partial withdrawal of the sheath 21, is then deployed. The third graft 10b positioned on the third balloon catheter is thus urged at its upstream end into contact with first tubular extension 19a and at its downstream end into contact with the right iliac artery 12 (see Fig. 7h).

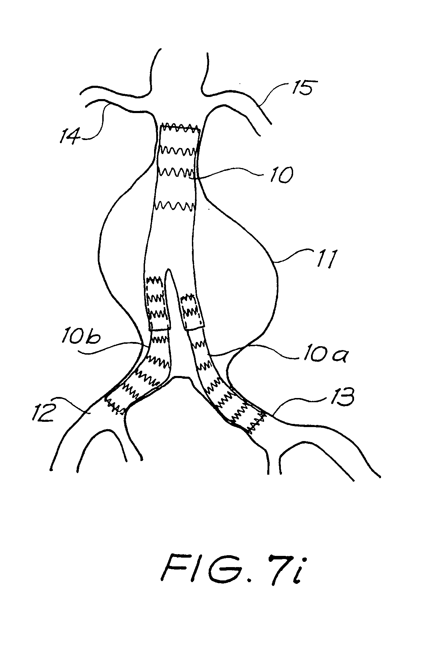

The stiff guidewire 23 and 30 are now withdrawn and the contralateral incision or puncture sutured. A second angiographic examination is undertaken and, if the grafts 10, 10a and 10b are correctly placed and functioning, the haemostatic sheath 21 is withdrawn and the right femoral incision or puncture sutured. The result is a functioning trouser graft bridging an aneurysm such as is depicted in Fig. 7i.

The operation may be carried out using a general anaesthetic, an epidural anaesthetic or, in suitable cases, using only a local anaesthetic.

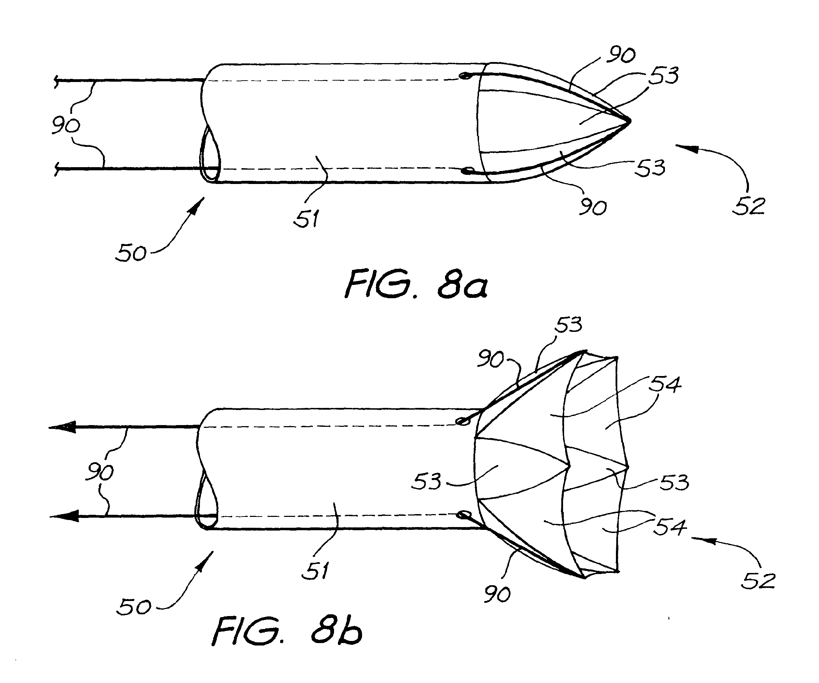

Figs 8a and 8b show the first end of a catheter 51 forming part of a guidewire capture sheath 50 according to the present invention. The catheter 51 has on its first end a capture means 52 comprising a number of petal like members 53 disposed around the circumference of the first end of the catheter 51. Each petal like member 53 is connected to its neighbouring petal like members 53 by a web 54 of a thin flexible film. The petal like members 53 are pivotable between a first position, in which they are relatively close together at their free ends, as are the petals in a flower bud, and a second position in which they are relatively spread apart, as in the manner of an opened flower. In the latter position, the petal like members 53 and the webs 54 together will form a funnel that will direct a guidewire into the lumen of the catheter 51. The petal like members 53 are caused to move between their first and second positions by a pair of wires 90 connected to the radially outer side of each of a pair of the petal like members 53, such that by pulling on the wires 90 the petal like members 58 are caused to be moved from their first position to their second position.

## Claims

1. A guidewire capture sheath for use in a bodily vessel comprising:-
(a) a catheter; and
(b) a capture means housed by, connected to, or integral with the catheter and being permanently disposed to project from one end of the catheter; at least a portion of the capture means being capable of expanding and collapsing between a first condition and at least a second condition, and wherein when in the first condition, at least a portion of the capture means forms a substantially conical nose on the catheter, said nose capable of assisting the passage of the catheter through the bodily vessel, wherein when in the second condition, the capture means is adapted to guide a guidewire into the catheter.

2. The guidewire capture sheath according to claim 1, wherein when in the first condition, at least a portion of the capture means is contracted to a cross-sectional area that is relatively smaller than that of the catheter, and when in the second condition, the portion of the capture means is expanded to a cross-sectional area that is relatively larger than that of the catheter.

3. The guidewire capture sheath according to claim 1 or 2, wherein when in the first condition, at least a portion of the capture means is disposed within the catheter, and when in the second condition, the portion of the capture means projects from an end of the catheter.

4. The guidewire capture sheath according to any one of the preceding claims wherein the capture means further comprises a guidewire following aperture adapted to relatively slide over a guidewire positioned in the bodily vessel.

5. The guidewire capture sheath according to any one of the preceding claims, wherein once the guidewire capture sheath is in the desired location, the at least one portion of the capture means is caused or allowed to expand to its second condition.

6. The guidewire capture sheath according to any one of the preceding claims, wherein when in the second condition, an outer perimeter of the at least one portion of the capture means bears against the luminal wall of the vessel in which the guidewire capture sheath has been deployed.

7. The guidewire capture sheath according to claim 1, wherein the at least one portion of the capture means further comprises a plurality of petal members each having a first free end and a second end which is connected to one end of the catheter, said petal members each being pivotable about the connection with the catheter between a first position, in which they are relatively close together at their free ends, and a second position, in which they are relatively spread apart.

8. The guidewire capture sheath according to claim 7, wherein each petal member is connected to its neighbouring petal member by a web of a thin flexible film.

9. The guidewire capture sheath according to claim 7 or 8, wherein when in the second position, the petal members and the web form a funnel which is directed into the lumen of the catheter.

10. The guidewire capture sheath according to any one of claims 7 to 9, wherein the capture means further comprises a wire or wires connected to at least one of the petal members, such that on manipulation of the wire or wires, the petal members are made to move between their respective first and second positions.

11. The guidewire capture sheath according to any one of claims 7 to 9, wherein the at least one portion of the capture means is comprised of two petal members and a push tube disposed within the catheter, such that the free ends of the petal members are superposed, and the second end of each petal member is respectively connected to one end of the catheter and one end of the push tube; the capture means being such that upon relative movement between the catheter and the push tube, the petal members are caused to move between their first and second positions.

12. The guidewire capture sheath according to any one of claims 7 to 9, wherein the petal members are allowed to change from their first position to their second position by memory-aided change.

13. The guidewire capture sheath according to claim 12. wherein the capture means is manufactured such that the petal members are initially in their respective second positions.

14. The guidewire capture sheath according to claim 13, wherein manual compression is adequate to cause the petal members to change from their respective second positions, said manual compression being only adequate to cause the petal members to undergo said change only temporarily, such that upon release of said manual compression, the petal members immediately return to their respective second positions.

15. The guidewire capture sheath according to any one of claims 7 to 9, wherein the petal members are allowed to change from their first position to their second position by heat-aided change.

16. The guidewire capture sheath according to claim 15, wherein the introduction of the guidewire catheter sheath into a vessel in the body of a patient is adequate to change its temperature sufficiently to cause the petal members to change from their respective first positions to their respective second positions.

17. The guidewire capture sheath according to any one of claims 7 to 16, wherein at least one of the petal members are semi-permeable.

## Patentansprüche

1. Ummantelung oder Einrichtung zum Halten oder Fangen eines Führungsdrahtes in einem Körpergefäß mit
a) einem Katheter und
b) einem Mittel zum Fangen oder Halten, welches aufgenommen ist in, verbunden ist mit oder integral ausgebildet ist mit dem Katheter und permanent oder dauerhaft geeignet angeordnet ist, um aus einem Ende des Katheters herauszustehen, wobei zumindest ein Teil des Mittels zum Halten oder Fangen expandiert und zusammengefaltet werden kann zwischen einem ersten Zustand und zumindest einem zweiten Zustand und wobei in dem ersten Zustand zumindest ein Teil des haltenden oder fangenden Mittels eine im Wesentlichen konische Nase auf oder an dem Katheter bildet, die den Durchtritt des Katheters durch das Körpergefäß unterstützt, und wobei in dem zweiten Zustand das haltende oder fangende Mittel eine Führung eines Führungsdrahts in den Katheter ermöglicht.

2. Ummantelung oder Einrichtung nach Anspruch 1, **dadurch gekennzeichnet, dass** in dem ersten Zustand zumindest ein Teil des haltenden oder fangenden Mittels zusammengezogen oder verkleinert ist auf eine Querschnittsfläche, die kleiner ist als die des Katheters, und im zweiten Zustand der Teil des haltenden oder fangenden Mittels expandiert ist auf eine Querschnittsfläche, die größer ist als die des Katheters.

3. Ummantelung oder Einrichtung nach Anspruch 1 oder 2, **dadurch gekennzeichnet, dass** in dem ersten Zustand zumindest ein Teil des haltenden oder fangenden Mittels in dem Katheter angeordnet ist und in dem zweiten Zustand der Teil des haltenden oder fangenden Mittels aus einem Endbereich des Katheters hervorsteht.

4. Ummantelung oder Einrichtung nach einem der vorhergehenden Ansprüche, **dadurch gekennzeichnet, dass** das haltende oder fangende Mittel eine dem Führungsdraht folgende Öffnung besitzt, die geeignet gestaltet ist, um ein relatives Gleiten über einen Führungsdraht, der in dem Körpergefäß angeordnet ist, zu ermöglichen.

5. Ummantelung oder Einrichtung nach einem der vorhergehenden Ansprüche, **dadurch gekennzeichnet, dass**, wenn die Ummantelung oder Einrichtung einmal an dem gewünschten Ort ist, zumindest ein Teil der Ummantelung oder der Einrichtung expandieren kann in den zweiten Zustand oder eine derartige Expansion verursacht wird.

6. Ummantelung oder Einrichtung nach einem der vorhergehenden Ansprüche, **dadurch gekennzeichnet, dass** in dem zweiten Zustand ein äußerer Umfang des zumindest einen Teiles der Ummantelung oder der Einrichtung abgestützt ist gegen die luminale Wandung des Gefäßes, in das die Ummantelung oder die Einrichtung eingesetzt ist.

7. Ummantelung oder Einrichtung nach Anspruch 1, **dadurch gekennzeichnet, dass** der zumindest eine Teil des haltenden oder fangenden Mittels mehrere Verschlusselemente oder blütenartige Elemente besitzt, die jeweils ein erstes freies Ende und ein zweites Ende besitzen, welches mit einem Ende des Katheters verbunden ist, wobei die Verschlusselemente oder blütenartigen Elemente jeweils um den Verbindungsbereich mit dem Katheter verschwenkbar sind zwischen einer ersten Position, in der diese an ihren freien Enden verhältnismäßig dicht zusammen sind, und einer zweiten Position, in der diese verhältnismäßig weit voneinander entfernt sind.

8. Ummantelung oder Einrichtung nach Anspruch 7, **dadurch gekennzeichnet, dass** jedes blütenartige Element oder Verschlusselement mit dem benachbarten blütenartigen Element oder Verschlusselement verbunden ist durch ein Gewebe oder Netz eines dünnen flexiblen filmartigen Materials.

9. Ummantelung oder Einrichtung nach Anspruch 7 oder 8, **dadurch gekennzeichnet, dass** in der zweiten Position die blütenartige Elemente oder Verschlusselemente und das Gewebe oder Netz einen Trichter bilden, der in das Lumen des Katheters gerichtet ist.

10. Ummantelung oder Einrichtung nach einem der Ansprüche 7 bis 9, **dadurch gekennzeichnet, dass** die Ummantelung oder die Einrichtung einen Draht oder mehrere Drähte besitzt, der oder die mit dem zumindest einen blütenartigen Element oder Verschlusselement verbunden ist/sind, so dass mit Manipulation des mindestens einen Drahtes eine Bewegung der blütenartigen Elemente oder Verschlusselemente zwischen der ersten und zweiten Position verursacht wird.

11. Ummantelung oder Einrichtung nach einem der Ansprüche 7 bis 9, **dadurch gekennzeichnet, dass** zumindest ein Teil der Ummantelung oder der Einrichtung mit zwei blütenartigen Elementen oder Verschlusselementen gebildet ist und ein Druckrohr in dem Katheter angeordnet ist, wobei die freien Enden der blütenartigen Elemente oder Verschlusselemente überlagert sind, und dass das zweite Ende von jedem blütenartigen Element oder Verschlusselement mit einem Ende des Katheters und einem Ende des Druckrohres verbunden ist und dass das haltende oder fangende Mittel derart ausgebildet ist, dass mit einer Relativbewegung zwischen dem Katheter und dem Druckrohr eine Bewegung der blütenartigen Elemente oder Verschlusselemente zwischen der ersten und zweiten Position verursacht wird.

12. Ummantelung oder Einrichtung nach einem der Ansprüche 7 bis 9, **dadurch gekennzeichnet, dass** die blütenartigen Elemente oder Verschlusselemente Memory-unterstützt ihre Position ändern können von der ersten Position zu der zweiten Position.

13. Ummantelung oder Einrichtung nach Anspruch 12, **dadurch gekennzeichnet, dass** das haltende oder fangende Mittel derart hergestellt ist, dass sich die blütenartigen Elemente oder Verschlusselemente anfänglich in ihren zweiten Positionen befinden.

14. Ummantelung oder Einrichtung nach Anspruch 13, **dadurch gekennzeichnet, dass** die manuelle Kompression geeignet ist, um eine Veränderung der Position der blütenartigen Elemente oder Verschlusselemente von ihren zweiten Positionen zu verursachen, wobei die manuelle Kompression lediglich geeignet ist zur temporären Hervorrufung der Veränderung der blütenartigen Elemente oder Verschlusselemente, so dass mit einer Freigabe der manuellen Kompression die blütenartigen Elemente oder Verschlusselemente unverzüglich wieder in ihre jeweilige zweite Position zurückkehren.

15. Ummantelung oder Einrichtung nach einem der Ansprüche 7 bis 9, **dadurch gekennzeichnet, dass** die blütenartigen Elemente oder Verschlusselemente ihre Position von der ersten Position zu der zweiten Position mit einer Wärme- oder Hitze-unterstützten Veränderung verändern können.

16. Ummantelung oder Einrichtung nach Anspruch 15, **dadurch gekennzeichnet, dass** das Einführen der Ummantelung oder Einrichtung in ein Gefäß in dem Körper eines Patienten geeignet ist zur Veränderung der Temperatur in einem Ausmaß, welches ausreichend ist um zu verursachen, dass die blütenartigen Elemente oder Verschlusselemente ihre Position verändern von der ersten Position in die zweite Position.

17. Ummantelung oder Einrichtung nach einem der Ansprüche 7 bis 16, **dadurch gekennzeichnet, dass** zumindest eines der blütenartigen Elemente oder Verschlusselemente semipermeabel ist.

## Revendications

1. Gaine de capture de fil-guide destinée à être utilisée dans un vaisseau corporel comprenait
(a) un cathéter ; et
(b) des moyens de capture logés dans, reliés au, ou d'un seul tenant avec le cathéter et disposés de manière permanente de façon à faire saillie d'une extrémité du cathéter, au moins une partie des moyens de capture étant capable de s'élargir et de se rétracter entre un premier état et au moins un deuxième état, et dans laquelle, dans le premier état, au moins une partie des moyens de capture forme un nez sensiblement conique sur le cathéter, ledit nez étant capable d'aider au passage du cathéter à travers le vaisseau corporel, et dans laquelle, dans le deuxième état, les moyens de capture sont adaptés pour guider un fil-guide dans le cathéter.

2. Gaine de capture de fil-guide selon la revendication 1, dans laquelle, dans le premier état, au moins une partie des moyens de capture est contractée en une section transversale qui est relativement plus petite que celle du cathéter et, dans le deuxième état, la partie des moyens de capture est élargie en une section transversale qui est relativement plus grande que celle du cathéter.

3. Gaine de capture de fil-guide selon la revendication 1 ou 2, dans laquelle, dans le premier état, au moins une partie des moyens de capture est disposée dans le cathéter et, dans le deuxième état, la partie des moyens de capture fait saillie d'une extrémité du cathéter.

4. Gaine de capture de fil-guide selon l'une quelconque des revendications précédentes, dans laquelle les moyens de capture comprennent en outre une ouverture de suivi de fil-guide adaptée pour coulisser de manière relative sur un fil-guide positionné dans le vaisseau corporel.

5. Gaine de capture de fil-guide selon l'une quelconque des revendications précédentes, dans laquelle, une fois que la gaine de capture de fil-guide est à l'emplacement souhaité, ladite au moins une partie des moyens de capture est amenée ou autorisée à s'élargir dans son deuxième état.

6. Gaine de capture de fil-guide selon l'une quelconque des revendications précédentes, dans laquelle, dans le deuxième état, un périmètre extérieur de ladite au moins une partie des moyens de capture s'appuie contre la paroi luminale du vaisseau dans lequel la gaine de capture de fil-guide a été déployée.

7. Gaine de capture de fil-guide selon la revendication 1, dans laquelle ladite au moins une partie des moyens de capture comprend en outre une pluralité d'éléments en forme de pétales ayant chacun une première extrémité libre et une deuxième extrémité qui est reliée à une extrémité du cathéter, lesdits éléments en forme de pétales étant capables chacun de pivoter autour de la liaison avec le cathéter entre une première position, dans laquelle ils sont relativement proches les uns des autres à leurs extrémités libres, et une deuxième position, dans laquelle ils sont relativement étalés et espacés.

8. Gaine de capture de fil-guide selon la revendication 7, dans laquelle chaque élément en forme de pétale est relié à son élément en forme de pétale voisin par une bande d'un mince film souple.

9. Gaine de capture de fil-guide selon la revendication 7 ou 8, dans laquelle, dans la deuxième position, les éléments en forme de pétales et la bande forment un entonnoir qui est dirigé dans la lumière du cathéter.

10. Gaine de capture de fil-guide selon l'une quelconque des revendications 7 à 9, dans laquelle les moyens de capture comprennent en outre un fil ou des fils reliés à au moins l'un des éléments en forme de pétales, de sorte que, lors de la manipulation du fil ou des fils, les éléments en forme de pétales sont amenés à se déplacer entre leurs premières et deuxièmes positions respectives.

11. Gaine de capture de fil-guide selon l'une quelconque des revendications 7 à 9, dans laquelle ladite au moins une partie des moyens de capture est composée de deux éléments en forme de pétales et d'un tube de poussée disposé dans le cathéter, de sorte que les extrémités libres des éléments en forme de pétales sont superposées, et la deuxième extrémité de chaque élément en forme de pétale est respectivement reliée à une extrémité du cathéter et à une extrémité du tube de poussée ; les moyens de capture étant tels que, lors d'un mouvement relatif entre le cathéter et le tube de poussée, les éléments en forme de pétales sont amenés à se déplacer entre leurs première et deuxième positions.

12. Gaine de capture de fil-guide selon l'une quelconque des revendications 7 à 9, dans laquelle les éléments en forme de pétales sont autorisés à passer de leur première position à leur deuxième position par un changement assisté par mémoire de forme.

13. Gaine de capture de fil-guide selon la revendication 12, dans laquelle les moyens de capture sont fabriqués de sorte que les éléments en forme de pétales soient initialement dans leurs deuxièmes positions respectives.

14. Gaine de capture de fil-guide selon la revendication 13, dans laquelle une compression manuelle est adéquate pour amener les éléments en forme de pétales à changer de position à partir de leurs deuxièmes positions respectives, ladite compression manuelle n'étant adéquate que pour amener les éléments en forme de pétales à subir ledit changement seulement temporairement, de sorte que, lors du relâchement de ladite compression manuelle, les éléments en forme de pétales retournent immédiatement à leurs deuxièmes positions respectives.

15. Gaine de capture de fil-guide selon l'une quelconque des revendications 7 à 9, dans laquelle les éléments en forme de pétales sont autorisés à passer de leur première position à leur deuxième position par un changement assisté par la chaleur.

16. Gaine de capture de fil-guide selon la revendication 15, dans laquelle l'introduction de la gaine à cathéter à fil-guide dans un vaisseau dans le corps d'un patient est adéquate pour modifier sa température suffisamment pour amener les éléments en forme de pétales à passer de leurs premières positions respectives à leurs deuxièmes positions respectives.

17. Gaine de capture de fil-guide selon l'une quelconque des revendications 7 à 16, dans laquelle au moins l'un des éléments en forme de pétales est semi-perméable.
